# EUROPÄISCHE PATENTANMELDUNG

(11) **EP 1 518 565 A1**
(43) Veröffentlichungstag der Anmeldung: **30.03.2005**
(21) Anmeldenummer: 04000715.5
(22) Anmeldetag: 15.01.2004
(51) Int. Cl.: A61L 2/10, A61L 2/20, B65B 55/08, B65B 55/10, B67B 1/03, B67B 3/06, A61L 2/26

(54) **Vorrichtung zur Entkeimung von Behälterverschlüssen**

(30) Priorität: 26.09.2003 DE 20314955 U
(71) Anmelder: Geiser, Alain, 79112 Freiburg i.B. (DE)
(72) Erfinder: Geiser, Alain, 79112 Freiburg i.B. (DE)
(74) Vertreter: Blum, Rudolf E.

(57) **Zusammenfassung**

Ein schräggestelltes, als Klappenrutsche ausgebildetes Verschlussdurchlaufgehäuse (10) ist in mehrere Kammern (2-9) aufgeteilt, die durch Klappen (11-17) voneinander getrennt sind. Eintrittsseitig befindet sich eine Verschlusseinlaufkammer (2) und austrittsseitig befindet sich eine Verschlussaustrittskammer (9). Die Verschlusseinlaufkammer (2) ist über eine luftdicht verschliessende Klappe (11) gegen die ihr folgende Schleusenkammer (3) absperrbar, welche ihrerseits über eine weitere luftdicht schliessende Klappe (12) gegen die ihr folgende erste Entkeimungskammer (4) absperrbar ist. Diese erste Entkeimungskammer (4) ist von weiteren Entkeimungskammern (5-7) gefolgt, die gegen einander durch luftdurchlässig schliessende Klappen (13-25) abgegrenzt sind. Die unterste Entkeimungskammer (7) ist von einer weiteren Schleusenkammer (8) gefolgt und über eine luftdicht schliessende Klappe (16) von derselben getrennt. Die Schleusenkammer (8) ist schliesslich von einer Verschlussaustrittskammer (9) gefolgt, und ebenfalls über eine luftdicht schliessende Klappe (17) von derselben getrennt. Über den Entkeimungskammern (5-7) ist eine UV-Strahlvorrichtung (19) angeordnet. Die Entkeimungskammern (5-7) werden weiter durch Ozon beaufschlagt, wobei eine Ozonvernichtungsvorrichtung (21) vorhanden ist, so dass die Schleusenkammern (3,8) taktweise durchspülbar sind.

## Beschreibung

Die vorliegende Erfindung betrifft eine Vorrichtung zur Entkeimung von Behälterverschlüssen.

Verkeimte Oberflächen allgemein von Behältern und Verschlüssen stellen ein erhebliches Risiko für die Qualität jedes mit solchen Behältern und Verschlüssen in Berührung kommenden Produktes, insbesondere eines verzehrbaren Produktes dar. Abhängig von einem jeweiligen Einfüllgut und der Dauer der beabsichtigten Haltbarkeit, und offensichtlich auch hinsichtlich Krankheitserregern, ist auf die Entkeimung, die Desinfizierung eines jeweiligen Behälters und des Behälterverschlusses ein besonderes Augenmerk zu richten.

Auch bei neu hergestellten und einem Abfüllbetrieb zugelieferten und dort gelagerten Behälterverschlüssen wie Flaschenverschlüsse besteht die Gefahr der Verkeimung. Die Umgebungsluft während dem Antransport und der Lagerung von Behälterverschlüssen, beispielsweise Flaschenverschlüssen, Sportverschlüssen und Kronkorken, führt bekanntlicherweise oft zu einer Verkeimung der Verschlüsse, die die Produktqualität gefährdet und die Haltbarkeit beeinträchtigt.

Dieses Problem tritt vermehrt insbesondere bei Flaschen auf, deren Einfüllgut ein sogenanntes stilles Wasser ist.

Es sind schon verschiedene Vorrichtungen und Vorgehensweisen zur Entkeimung von Flaschenverschlüssen bekannt geworden, die jedoch nicht zu einer befriedigenden und ökonomischen Entkeimung derselben führen.

Beispielsweise ist bedingt durch die Form der Verschlüsse sowie die Gewindegänge an der Innenseite der Verschlüsse eine reine UV-Entkeimung aufgrund des Schatteneffektes nicht ausreichend.

Weiter sind Vorrichtungen bekannt geworden, in welchen Flaschenverschlüsse mittels wässriger Medien chemisch behandelt worden, beispielsweise mittels einer Zufuhr von H₂O₂, das mit Wasser gemischt dampfförmig auf die Flaschenverschlüsse aufgebracht wird. Dieses bedingt jedoch eine zusätzliche Apparatur zum Trocknen der entsprechend entkeimten Flaschenverschlüsse.

Auch bekannt sind Vorrichtungen, mittels welchen eine Sterilisation mittels einer Peressigsäure, gefolgt von einem Abspülen mittels Sterilwasser und einem Trocknen mit Sterilluft erfolgt. Auch diese Vorrichtungen sind äusserst aufwendig.

Hier will die Erfindung Abhilfe schaffen. Die Erfindung, wie sie in den Ansprüchen gekennzeichnet ist, löst die Aufgabe, eine Vorrichtung zur Entkeimung von Flaschen zu schaffen, mittels welcher keine Beeinträchtigung der Entkeimungswirkung durch einen Schatteneffekt auftritt und die mit einem trockenen Entkeimen arbeitet.

Die erfindungsgemässe Vorrichtung ist gekennzeichnet durch ein langgestrecktes, schräggestelltes und in mehrere Kammern unterteiltes Verschlussdurchlaufgehäuse, welche Kammern mittels Klappen gegeneinander abgegrenzt sind, durch eine mit einer Anzahl der Kammern lichtseitig in Verbindung stehende UV-Strahlvorrichtung, und durch eine Ozonvorrichtung mit einer mit einer dieser Kammern gasseitig in Verbindung stehenden Ozonerzeugervorrichtung und mit einer Ozonvernichtungsvorrichtung.

Vorteilhafte Ausbildungen der erfindungsgemässen Vorrichtung ergeben sich aus den abhängigen Ansprüchen.

Die durch die Erfindung erreichten Vorteile sind im wesentlichen darin zu sehen, dass die energiereiche UV-Strahlung in Kombination mit Photo-Ozon vorteilhaft eine vollständige Abtötung von an den Verschlüssen haftenden Keimen sichert, ohne ein jeweiliges Produkt zu gefährden. Ein nachfolgendes Trocknen der Verschlüsse entfällt.

Im folgenden wird die Erfindung anhand von lediglich einen Ausführungsweg darstellenden Zeichnungen näher erläutert.

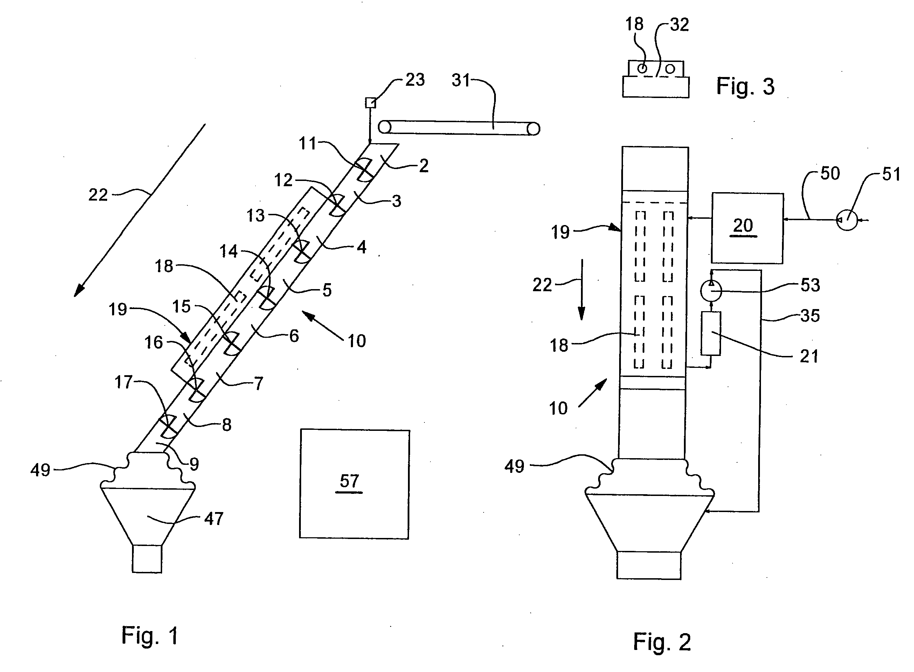

Abbildung 1 zeigt vereinfacht eine als Klappenrutsche ausgebildete Ausführung der erfindungsgemässen Vorrichtung,
Abbildung 2 zeigt eine Seitenansicht der Klappenrutsche nach Abbildung 1 mit zusätzlichen, schematisch dargestellten Vorrichtungskomponenten,
Abbildung 3 zeigt eine Aufsicht auf die Klappenrutsche nach Abbildung 1,
Abbildung 4 zeigt schematisch die gesamte Anlage mit dem Leitungsschema, und
Abbildung 5 zeigt in vergrössertem Massstab die Klappenrutsche nach Abbildung 1 in einer mehr detaillierten Darstellung.

Die Klappenrutsche weist ein langgestrecktes Verschlussdurchlaufgehäuse 10 auf. Dieses Verschlussdurchlaufgehäuse 10 ist schräggestellt, so dass zu entkeimende Flaschenverschlüsse aufgrund der Gravitationskraft das Verschlussdurchlaufgehäuse 10 durchlaufen können. Die Neigung der Klappenrutsche beträgt 45°-60°.

Oberhalb des Verschlussdurchlaufgehäuses 10 ist ein Zufuhrförderer 31 angeordnet. Die zu entkeimenden Verschlüsse werden von einer Lagerstelle des jeweiligen Betriebes mittels dem Zufuhrförderer 31 zum Verschlussdurchlaufgehäuse 10 gefördert. Dieser Zufuhrförderer 31 kann, wie gezeigt, ein Transportband aufweisen, kann jedoch auch beispielsweise als Druckluftförderer ausgebildet sein.

Das Verschlussdurchlaufgehäuse 10 ist in mehrere Kammern unterteilt. Die gravitationskraftbedingte Verschlussdurchlaufrichtung durch das Verschlussdurchlaufgehäuse ist mittels dem Pfeil 22 aufgezeigt.

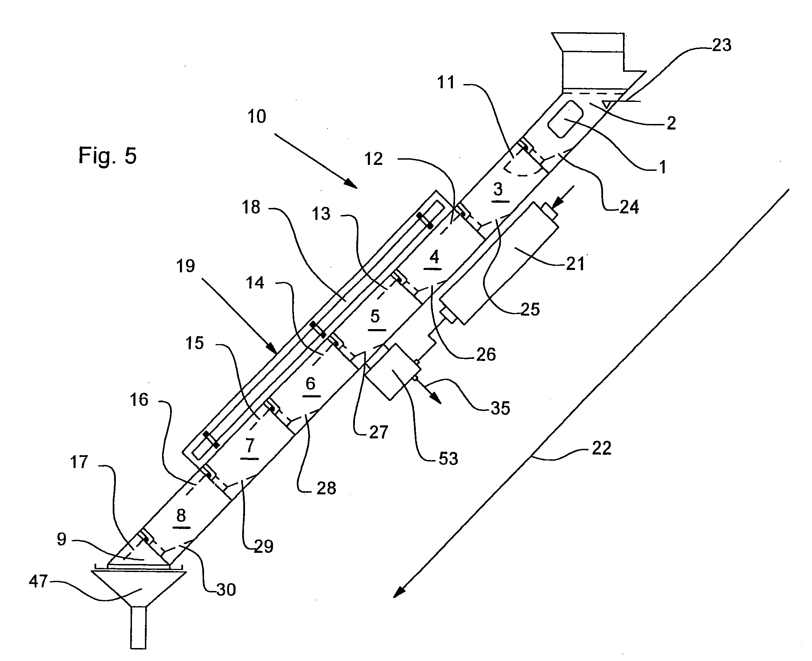

Unmittelbar unter dem Ende des Verschlusszufuhrförderers 31 ist als erste Kammer des Verschlussdurchlaufgehäuses 10 eine Verschlusseinlaufkammer 2 mit einem Sichtfenster 1 (siehe Fig. 5) angeordnet. Diese Verschlusseinlaufkammer 2 weist eine Mengenfühlervorrichtung 23 auf, beispielsweise ein kapazitiver Näherungsschalter, der dazu dient, eine Vollmeldung der Verschlusseinlaufkammer 2 an die Steuerung der Vorrichtung abzugeben.

Die Verschlussaufnahmekammer 2 ist in Verschlussdurchlaufrichtung 22 von einer ersten Schleusenkammer 3 gefolgt, die als Einlaufschleuse dient.

An der ersten Schleusenkammer 3 schliessen vier aufeinanderfolgend angeordnete Entkeimungskammern 4, 5, 6, 7 an. Die Anzahl Entkeimungskammern 4, 5, 6, 7 ist lediglich als Beispiel zu betrachten. Sie richtet sich je nach Ausführung nach der Verschlussentkeimungsleistung und der Verschlussart.

Auf diesen Entkeimungskammern folgt eine zweite Schleusenkammer 8, die als Auslaufschleuse dient.

Schliesslich ist eine mit dem Einlauftrichter 47 der Behälter-, bzw. Flaschenverschliessvorrichtung 48 dicht verbundene Verschlussaustrittskammer 9 vorhanden. Abhängig von örtlichen Gegebenheiten ist das Verschlussdurchlaufgehäuse 10 fest oder, wie gezeichnet, durch einen elastischen Abdichtungsschlauch 49 mit dem Einlauftrichter 47 verbunden.

Die einzelnen Kammern sind mittels Klappen mit einem jeweiligen (nicht gezeichneten) Schwenkantrieb wie folgt gegeneinander abgegrenzt.

Die Verschlussaufnahmekammer 2 ist mittels einer ersten Klappe 11 gegenüber der ersten Schleusenkammer 3 luftdicht abtrennbar, das heisst, die erste Klappe 11 ist im geschlossenen Zustand gegenüber dem Vorrichtungsgehäuse abgedichtet.

Weiter ist die erste Schleusenkammer 3 mittels einer weiteren Klappe 12 gegenüber der ihr unmittelbar nachfolgenden Entkeimungskammer 4 luftdicht abtrennbar. Somit ist die erste Schleusenkammer 3 im geschlossenen Zustand der Klappen 11 und 12 vollständig abgedichtet.

Die zweite Schleusenkammer 8 ist mittels einer zweiten Klappe 18 gegenüber der Verschlussaustrittskammer 9 luftdicht abtrennbar, das heisst, die zweite Klappe 18 ist im geschlossenen Zustand gegenüber dem Vorrichtungsgehäuse abgedichtet.

Die zweite Schleusenkammer 8 ist mittels einer zweiten Klappe 18 gegenüber der Verschlussaustrittskammer 9 luftdicht abtrennbar, das heisst, die zweite Klappe 18 ist im geschlossenen Zustand gegenüber dem Vorrichtungsgehäuse abgedichtet.

Weiter ist die zweite Schleusenkammer 8 mittels einer noch weiteren Klappe 17 gegenüber der ihr unmittelbar vorausgehenden Entkeimungskammer 7 luftdicht abtrennbar. Somit ist die zweite Schleusenkammer 8 ebenfalls im geschlossenen Zustand der Klappen 17 und 18 vollständig abgedichtet.

Die Klappen 13, 14, 15, welche die vier Entkeimungskammern 4, 5, 6, 7 gegeneinander abgrenzen, sind im geschlossenen Zustand gegenüber dem Vorrichtungsgehäuse nicht abgedichtet, so dass bei geschlossenen Klappen 13, 14, 15 eine Luftdurchlässigkeit zwischen den Entkeimungskammern 4, 5, 6, 7 vorhanden ist, so dass, wie noch gezeigt sein wird, ein Ozonfluss von der ersten bis zur letzten Entkeimungskammer ermöglicht ist.

Sämtliche Klappen sind mit einem pneumatischen Schwenkantrieb ausgerüstet. Diese pneumatischen Schwenkantriebe sind weiter mit einer Endlager-Abfrage mittels pneumatischen bzw. elektrischen Ausgangssignalen ausgerüstet.

Die Kammern weisen Auflauframpen 24, 25, 26, 27, 28, 29, 30 auf. Diese unterstützen bei jeweils geöffneten Klappen während der gravitationsbedingten Abwärtsbewegung der Behälterverschlüsse jeweilige Lageveränderungen, also Veränderungen der räumlichen Orientierung und Mischbewegungen, so dass sichergestellt ist, dass jeder Oberflächenabschnitt eines jeweiligen Behälterverschlusses der Entkeimungswirkung ausgesetzt wird.

Auf dem Durchlaufgehäuse 10 ist eine UV-Strahlvorrichtung 19 mit UV-Niederdruckstrahlen 18 angeordnet.

Bei der gezeigten Ausführung sind die Strahlen 18 in 2er-Batterien angeordnet, wobei die Strahler durch Quarzglasröhren geschützt sind. Diese Strahler 18 sind in 2er-Batterien mit jeweils zwei Aufnahmeblöcken von der noch zu erläuternden ozonhaltigen Luft abgeschirmt.

Die Stärke und Anzahl der UV-Strahler sowie deren Anordnung richtet sich nach der Verschlussentkeimungsleistung und der Behälterverschlussart.

Zwischen der UV-Strahlvorrichtung 19 und den Entkeimungskammern 4, 5, 6, 7 ist ein UV-durchlässiges Gitter 32 oder eine UV-durchlässige Glasscheibe zum Schutz der sich in diesen Entkeimungskammern 4, 5, 6, 7 befindlichen Behälterverschlüssen angeordnet.

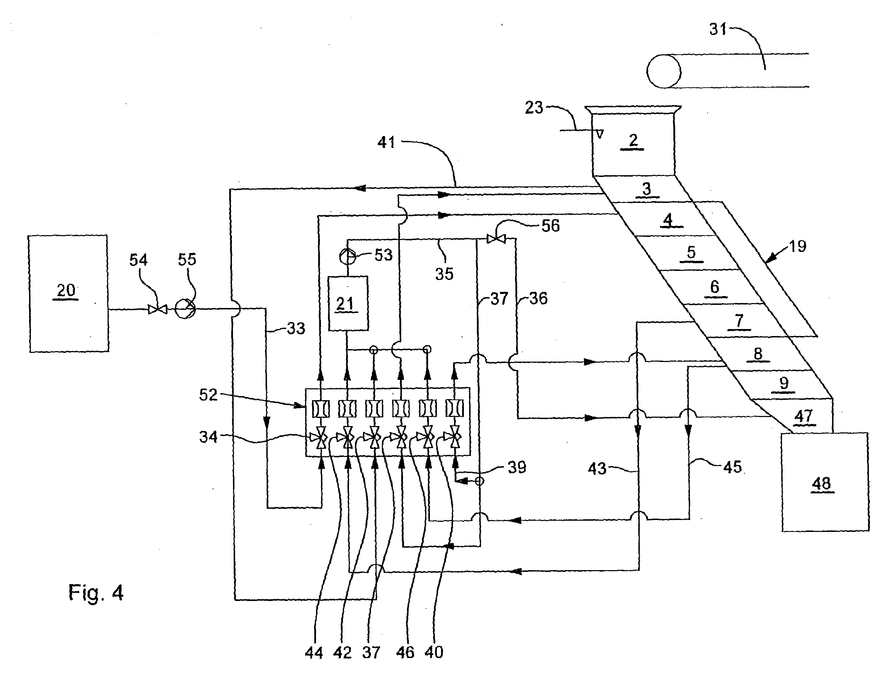

Nachfolgend wird nun der Ozon- bzw. Luftkreislauf unter Bezugnahme insbesondere auf Fig. 4 beschrieben.

Die Ozonvorrichtung weist eine Ozonerzeugervorrichtung 20 auf. Frischluft wird der Ozonerzeugervorrichtung durch eine Frischluftzufuhrleitung 50 mit einer Pumpe 51 zugeführt. Die Ozonerzeugung erfolgt in bekannter Weise durch eine stille elektrische Entladung.

Von der Ozonerzeugervorrichtung 20 verläuft eine Ozonzufuhrleitung 33 zur ersten Entkeimungskammer 4. Dieser Ozonzufuhrleitung 33 ist eine erste Regelvorrichtung 34 mit einem Durchflussmesser und Steuerventil zugeordnet. Beim Austritt aus der Ozonerzeugervorrichtung 20 sind ein Schliessventil und eine Pumpe in der Leitung 33 angeordnet.

Diese und auch die nachfolgend beschriebenen Regelvorrichtungen sind in einem Durchfluss-Steuerschrank 52 angeordnet.

Weiter weist die Ozonvorrichtung eine Ozonvernichtungsvorrichtung 21 auf. Diese Ozonvernichtungsvorrichtung 21 weist eine katalytische Ozonvernichtungspatrone bekannter Bauart auf.

An dieser Ozonvernichtungsvorrichtung schliesst eine Reinluftleitung 35 mit einer Pumpe 53 an.

Die Reinluftleitung 35 weist einen ersten Leitungsarm 36 auf, der zum Einlauftrichter 47 unter der Verschlussaustrittskammer 9 verläuft, welcher Einlauftrichter 47 auf dem Behälterverschliessapparat 48 sitzt.

Im ersten Leitungsarm 36 ist ein Schliessventil 56 eingesetzt.

Weiter ist ein erster Kreislaufleitungsarm 37 mit den ersten Leitungsarm 36 verbunden, welcher erste Kreislaufleitungsarm 37 zur ersten Schleusenkammer 3 verläuft, welchem ersten Kreislaufleitungsarm 37 eine zweite Regelvorrichtung 38 im Steuerschrank 52 zugeordnet ist.

Von diesem ersten Kreislaufleitungsarm 37 ist ein zweiter Kreislaufleitungsarm 39 abgezweigt, der zur zweiten Schleusenkammer 8 verläuft und welchem eine dritte Regelvorrichtung 40 zugeordnet ist.

Von der ersten Schleusenkammer 3 verläuft eine erste Absaugleitung 41 zur Ozonvernichtungsvorrichtung 21, welcher ersten Absaugleitung 41 eine vierte Regelvorrichtung 42 zugeordnet ist.

Von der in Durchlaufrichtung 22 unmittelbar vor der zweiten Schleusenkammer 8 angeordneten Entkeimungskammer 7 verläuft eine zweite Absaugleitung 43 ebenfalls zur Ozonvernichtungsvorrichtung 21, wobei dieser zweiten Absaugleitung eine fünfte Regelvorrichtung 44 zugeordnet ist.

Schliesslich verläuft von der zweiten Schleusenkammer 8 eine dritte Absaugleitung 45 zur Ozonvernichtungsvorrichtung 21, welcher dritten Absaugleitung eine sechste Regelvorrichtung 46 zugeordnet ist.

Nachfolgend wird der Betrieb der Vorrichtung erläutert, wobei zu erwähnen ist, dass eine rein schematisch angedeutete zentrale SPS-Steuerung 57 zur Koordinierung und Automatisierung aller Vorgänge vorhanden ist.

Als Beispiel der zu entkeimenden Behälterverschlüsse sollen Flaschenverschlüsse mit Innengewinde angenommen werden, wobei das in den zu verschliessenden Flaschen angeordnete Gut sogenanntes stilles Wasser ist, welche bekanntlich, da es keine Kohlensäure enthält, sehr empfindlich gegen Keime, d.h. deren nachteiligen Wirkungen ist.

Die Verschlüsse werden nach Bedarf von einem Lager mittels dem Zufuhrförderer 31 zur Verschlusseinlaufkammer 2 geführt. Diese weist die Verschlussfühlervorrichtung 23, einen kapazitiven Näherungsschalter auf, der bei gefüllter Verschlusseinlaufkammer 2 eine Vollmeldung an das zentrale Steuergerät 57 gibt, womit der Zufuhrförderer 31 wieder stillgesetzt wird.

Danach wird die luftdichte Klappe 11 geöffnet, so dass die Verschlüsse in die erste Schleusenkammer 3 fallen. Ist die erste Schleusenkammer 3 gefüllt, schliesst die luftdichte Klappe 11. Als nächster Schritt wird die luftdichte Klappe 12 geöffnet, womit die Verschlüsse in die erste Entkeimungskammer 4 fallen.

Danach schliesst die luftdichte Klappe 12.

Dieser Entkeimungskammer 4 wird nun von der Ozonerzeugervorrichtung 20 her über die Ozonzufuhrleitung 33 Ozon zugeführt. Auch wird die UV-Strahlvorrichtung 19 eingeschaltet.

Durch die UV-Strahlen wird das O₃ zu O₂ + O⁻ umgesetzt, so dass eine äusserst starke und insbesondere schnelle Entkeimungswirkung erfolgt. Das heisst, die UV-Strahlen wirken als den Entkeimungsvorgang letztendlich beschleunigenden Katalysator.

Die Verweilzeit der Verschlüsse in dieser Kammer kann beispielsweise 5-10 Sekunden betragen.

Nach Ablauf der Verweilzeit wird die untere Klappe 13 der Entkeimungskammer 4 geöffnet, so dass die Verschlüsse in die Entkeimungskammer 5 fallen, in welcher die Entkeimung weiter stattfindet. Wie oben beschrieben, schliessen die Klappen 17, 14, 15 zwischen den Entkeimungskammern 4, 5, 6, 7 nicht luftdicht ab, so dass das in die Entkeimungskammer 4 einströmende Ozon auch in die nachfolgenden Entkeimungskammern 5, 6, 7 strömt.

Die genannte Klappe 13 schliesst, und nach Ablauf der Verweilzeit in der Entkeimungskammer 5 öffnet deren untere Klappe 14, womit die Verschlüsse zur weiteren Entkeimung in die Entkeimungskammer 6 fallen, und danach schliesst die Klappe 14.

Wieder nach Ablauf der Verweilzeit öffnet die Klappe 15, womit die Verschlüsse in die Entkeimungskammern 7 fallen. Bei gefüllter Entkeimungskammer 7 schliesst die Klappe 15.

Nach Ablauf der dortigen Verweilzeit öffnet die luftdichte Klappe 16, so dass die Verschlüsse in die zweite Schleusenkammer 8 fallen.

Aus dieser wird nun über die dritte Absaugleitung 45 das Ozon abgesaugt und der Ozonvernichtungsvorrichtung 21 zugeführt.

Von der Ozonvernichtungsvorrichtung strömt Reinluft über die Pumpe 53, Reinluftleitung 35, erster Kreislaufleitungsarm 37 und zweiter Kreislaufleitungsarm 39 zurück in.die zweite Schleusenkammer 8, so dass das Ozon aus derselben ausgespült wird.

Danach öffnet die luftdichte Klappe 17 der zweiten Schleusenkammer 8, so dass die Verschlüsse in die Austrittskammer 9 fallen.

Von der Austrittskammer 9 fallen die entkeimten Verschlüsse schlussendlich in den Einlauftrichter 47 der Flaschenverschliessvorrichtung, um auf den Flaschen aufgeschraubt zu werden.

Auch diesem Einlauftrichter 47 wird vor der Ozonvernichtungsvorrichtung 21 her über die Reinluftleitung 35 und dem Leitungsarm 36 gereinigte Luft zugeführt.

Beim Füllen der obersten Entkeimungskammer 4 vor der ersten Schleusenkammer 3 her bzw. dem Füllen der ersten Schleusenkammer 3 von der Einlaufkammer 2 darf ebenfalls kein Ozon in die Umgebung austreten.

Auch hier ist ein Spülkreislauf vorhanden.

Von der ersten Schleusenkammer 3 wird die ozonhaltige Luft durch die erst Absaugleitung 41 abgesogen und der Ozonvernichtungsvorrichtung 21 zugeführt, von dieser wird die Reinluft über die Reinluftleitung 35 und den ersten Kreislaufleitungsarm 37 wieder zur ersten Schleusenkammer 3 zugeführt.

Bei der obigen Beschreibung des Betriebes wurde aus Gründen der Klarheit der Durchgang einer einzigen Charge Verschlüsse beschrieben.

Im Dauerbetrieb jedoch ist in jeder Kammer eine Charge Verschlüsse angeordnet. Das heisst, dass nachdem der Flaschenverschlusseinrichtung 48 eine Charge Verschlüsse aus der Austrittskammer 9 zugeführt worden sind, sämtliche Klappen aufeinanderfolgend geöffnet und wieder geschlossen werden. Dabei ist zu bemerken, dass eine nächste Klappe nur dann öffnen kann, wenn die vorliegende Klappe wieder in der Schliessstellung wirkt. Das heisst, die Klappen werden kaskadenförmig nacheinander geöffnet und wieder verschlossen.

Zu erwähnen ist noch, dass der UV-Strahlvorrichtung in der Steuerung 57 ein Zeitglied zugeordnet ist, so dass bei längeren Pausen die UV-Strahlen automatisch abgeschaltet werden, um eine Erwärmung der Verschlüsse zu vermeiden.

In gleicher Weise ist auch der Ozonerzeugervorrichtung ein Zeitglied in der Steuerung 57 zugeordnet. Bei längeren Pausen wird die Ozonerzeugung mittels Ventil bzw. Pumpe ausgeschaltet; der Ozon-Erzeuger unterbricht dann seinen Betrieb.

## Patentansprüche

1. Vorrichtung zur Entkeimung von Behälterverschlüssen, **gekennzeichnet durch** ein langgestrecktes, schräggestelltes und in mehrere Kammern (2-9) unterteiltes Verschlussdurchlaufgehäuse (10), welche Kammern (2-9) mittels Klappen (10-18) gegeneinander abgegrenzt sind, **durch** eine eine Anzahl 4-7) der Kammern lichtseitig beaufschlagende UV-Strahlvorrichtung (19), und **durch** eine Ozonvorrichtung (20, 21) einschliesslich einer mit einer (4) dieser Kammern (2-9) gasseitig in Verbindung stehenden Ozonerzeugervorrichtung (20) und mit einer Ozonvernichtungsvorrichtung (21).

2. Vorrichtung nach Anspruch 1, **dadurch gekennzeichnet, dass** die Kammern (2-9) eine Verschlusseinlaufkammer (2), eine erste Schleusenkammer (3), eine Anzahl aufeinanderfolgende Entkeimungskammern (4, 5, 6, 7), eine zweite Schleusenkammer (8) und eine Verschlussaustrittskammer (9) umfassen, welche Kammern (2-9) in Verschlussdurchlaufrichtung (22) der zu entkeimenden Behälterverschlüsse aufeinanderfolgend angeordnet sind, dass die UV-Strahlvorrichtung (19) lichtseitig mit den Entkeimungskammern (4, 5, 6, 7) in Verbindung steht, und dass die Ozonerzeugervorrichtung (20) mit der in Verschlussdurchlaufrichtung (22) ersten Entkeimungskammer (4) in Verbindung steht.

3. Vorrichtung nach Anspruch 1 oder 2, **dadurch gekennzeichnet, dass** die Verschlusseinlaufkammer (2) eine Verschlussmengenfühlervorrichtung (23) aufweist und durch eine erste Klappe (11) gegenüber der ersten Schleusenkammer (3) luftdicht abschliessbar ist, welche erste Schleusenkammer (3) ihrerseits durch eine weitere Klappe (12) gegenüber der ihr folgenden Entkeimungskammer (4) luftdicht abschliessbar ist, dass die zweite Schleusenkammer (8) durch eine zweite Klappe (18) gegenüber der Verschlussaustrittskammer (9) luftdicht abschliessbar ist und durch eine noch weitere Klappe (17) gegenüber der ihr vorgeschalteten Entkeimungskammer (7) luftdicht abschliessbar ist, und dass die Entkeimungskammern (4, 5, 6, 7) über im Schliesszustand luftdurchlässigen Klappen (13, 14, 15) gegeneinander abgegrenzt sind.

4. Vorrichtung nach einem der vorangehenden Ansprüche, **dadurch gekennzeichnet, dass** mindestens die Entkeimungskammern (4, 5, 6, 7) austrittsseitig eine Auflauframpe (24-30) aufweisen, die dazu dient, den zu entkeimenden Behälterverschlüssen bei einer jeweils geöffneten Klappe (11-18) während ihrer gravitationsbedingten Abwärtsbewegung durch die betreffenden Kammern (2-7) eine Lageveränderung und Mischbewegung zu erteilen, um sicherzustellen, dass jeder Oberflächenabschnitt eines jeweiligen Behälterverschlusses der Entkeimungswirkung ausgesetzt wird.

5. Vorrichtung nach einem der vorangehenden Ansprüche, **gekennzeichnet durch** einen Verschlusszufuhrförderer (31), der bei der Verschlusseinlaufkammer (2) endet.

6. Vorrichtung nach einem der vorangehenden Ansprüche, **dadurch gekennzeichnet, dass** zwischen der UV-Strahlvorrichtung und den Entkeimungskammern (4-7) ein UV-durchlässiges Gitter (32) oder eine UV-durchlässige Glasscheibe zum Schutz der sich in den jeweiligen Entkeimungskammern (4-7) befindlichen Behälterverschlüssen angeordnet ist.

7. Vorrichtung nach einem der vorangehenden Ansprüche, **dadurch gekennzeichnet, dass** von der Ozonerzeugervorrichtung (20) eine Ozonzufuhrleitung (33) zur bezüglich der Verschlussdurchlaufrichtung (22) ersten Entkeimungskammer (4) verläuft, welcher Ozonzufuhrleitung (33) eine erste Regelvorrichtung (34) zugeordnet ist.

8. Vorrichtung nach einem der vorangehenden Ansprüche 2, **dadurch gekennzeichnet, dass** an der Ozonvernichtungsvorrichtung (21) eine Reinluftleitung (35) anschliesst, welche einen Leitungsarm (36) aufweist, der zu einem an der Verschlussaustrittskammer (9) anschliessenden Einlauftrichter (47) verläuft, der zur Verbindung mit einem Behälterverschliessapparat (48) bestimmt ist, und einen ersten Kreislaufleitungsarm (37) aufweist, der zur ersten Schleusenkammer (3) verläuft, und welchem eine zweite Regelvorrichtung (38) zugeordnet ist, von welchem ersten Kreislaufleitungsarm (37) seinerseits ein zweiter Kreislaufleitungsarm (39) abgezweigt ist, der zur zweiten Schleusenkammer (8) verläuft und welchem eine dritte Regelvorrichtung (40) zugeordnet ist.

9. Vorrichtung nach einem der vorangehenden Ansprüche, **dadurch gekennzeichnet, dass** von der ersten Schleusenkammer (3) eine erste Absaugleitung (41) zur Ozonvernichtungsvorrichtung (21) verläuft, welcher ersten Absaugleitung (41) eine vierte Regelvorrichtung (42) zugeordnet ist.

10. Vorrichtung nach einem der vorangehenden Ansprüche, **dadurch gekennzeichnet, dass** von der in Verschlussdurchlaufrichtung (22) unmittelbar vor der zweiten Schleusenkammer (8) angeordneten Entkeimungskammer (7) eine zweite Absaugleitung (43) zur Ozonvernichtungsvorrichtung (21) verläuft, welcher zweiten Absaugleitung (43) eine fünfte Regelvorrichtung (44) zugeordnet ist.

11. Vorrichtung nach einem der vorangehenden Ansprüche, **dadurch gekennzeichnet, dass** von der zweiten Schleusenkammer (8) eine dritte Absaugleitung (45) zur Ozonvernichtungsvorrichtung (21) verläuft, welcher dritten Absaugleitung (45) eine sechste Regelvorrichtung (46) zugeordnet ist.
